# EUROPEAN PATENT APPLICATION

(11) **EP 4 451 404 A2**
(43) Date of publication of application: **23.10.2024**
(21) Application number: 24171209.0
(22) Date of filing: 19.04.2024
(51) Int. Cl.: H01M 10/0525, H01M 10/054, H01M 10/0569, H01M 10/36, H01M 12/00

(54) **AN ELECTROLYTE SOLVENT WITH ADJUSTABLE SOLVATION PROPERTIES, PRODUCTION METHOD, AND APPLICATIONS THEREOF**

(30) Priority: 19.04.2023 TW 112114510
(71) Applicant: National Taiwan University of Science and Technology, 106 Taipei City (TW)
(72) Inventor: Hwang, Bing-Joe, 106 Taipei (TW); Mekonnen Tekaligne, Teshager, 106 Taipei (TW); Yang, Sheng-Chiang, 106 Taipei (TW); Su, Wei-Nien, 106 Taipei (TW)
(74) Representative: Cabinet Chaillot

(57) **Abstract**

The present invention provides an electrolyte solvent with adjustable solvation properties, and through an electrolyte technology, adjusts the anion-rich solvent with favorable solubility, and develops and synthesizes the solvent with weak solvation properties, so that the battery performance can be greatly improved and the solvent can be produced at low cost. The electrolyte technology of the present invention can control the electrochemical battery, taking the lithium battery as an example, the growth pattern of the lithium and the interface control of the positive and negative electrodes, which can help improve the safety of the lithium battery during fast charging, and it can be extended to various electrochemical devices such as metal/metal-ion/metal and metal-ion hybrid batteries.

## Description

### FIELD OF INVENTION

An electrolyte solvent, particularly an electrolyte solvent applied to electrochemical devices.

The electrolyte solvent described in the present invention is mainly used in various electrochemical batteries and will be described and illustrated below as the main application. However, the present invention is not limited to use in electrochemical batteries, but other possible and applicable electrochemical devices are covered by the present invention.

### BACKGROUND OF THE INVENTION

Lithium metal batteries (LMBs) have become an important research direction for next-generation electric vehicles and energy storage devices due to their high energy density. However, uncontrollable dendritic lithium growth, irreversibility of lithium layer peeling, and interfacial incompatibility, which rapidly lead to poor cycle life and reduced coulombic efficiency, have hindered the practical use of LMBs.

In conventional carbonic acid-based electrolyte solvents for LMBs, such as carbonate solvents, the lithium-ions can coordinate with the carbonate solvent molecules for solvation. However, too much free solvent in the carbonate solvent will form a solvent-separated ion pair (SSIP), which will form a poor solid electrolyte interface (SEI) with the organic oxide on the negative electrode surface, resulting in serious degradation of the subsequent LMBs performance. If the concentration of lithium salt is increased to reduce the free solvent, this will undoubtedly increase the cost of the electrolyte and cause additional problems associated with high electrolyte concentration.

In light of the above, there is an urgent need for a solution to the problem of electrical dissipation in electrochemical batteries caused by excessive free solvents in existing electrolyte solvents.

### SUMMARY OF THE INVENTION

In light of this, to solve the above problems and extend the cycle time of LMBs, the most efficient solution is to use electrolyte solvent technology to form a strong, uniform, and conductive SEI on the anode surface, which can potentially improve the electrochemical performance of LMBs, realize fast charging and wide operating temperature range, and meet various market demands in the future.

The present invention provides an electrolyte solvent with adjustable solvation properties comprising a chemical structure of Formula (1) as follows: 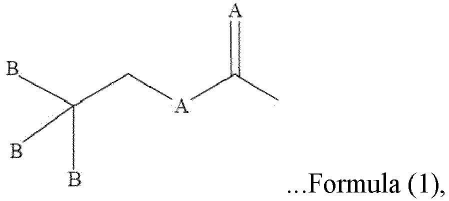 wherein A is oxygen (O), sulfur (S), selenium (Se), tellurium (Te), or combinations thereof; B comprises 1, 2 or 3 substituent(s) of halogen, nitrile (CN), hydrogen (H), nitro (NO₂), nitroso (NO), amine (NH₂), or combinations thereof.

The present invention also applies the electrolyte solvent with the aforementioned adjustable solvation properties to various electrochemical devices, such as various metal batteries.

From the above description, it is clear that the present invention has the following beneficial effects and advantages:
1. The present invention develops and synthesizes a solvent with weak solvation properties by electrolyte technology and adjusts anion-rich solvents with favorable solvation effect so that the battery performance is greatly improved and the solvent is of low production cost. The technology of the present invention can control the growth pattern of lithium and help improve the safety of lithium batteries during fast charging and can be extended to various electrochemical devices such as metal/metal-ion/metal and metal-ion hybrid batteries.
2. The electrolyte solvent provided in the present invention not only helps to deposit metal on the negative electrode surface and form a favorable solid electrolyte interface but also has a good interaction with the anion of metal salt and inhibits the formation of undesirable cathode electrode interface (CEI) at the positive electrode. It can significantly extend the life and safety of metal/metal-ion/metal-metal-ion hybrid batteries without increasing the metal salts' concentration, reducing the overall electrolyte cost.

Many of the attendant features and advantages of the present invention will become better understood with reference to the following detailed description considered in connection with the accompanying figures and drawings

### BRIEF DESCRIPTION OF THE DRAWINGS

The steps and the technical means adopted by the present invention to achieve the above and other objects can be best understood by referring to the following detailed description of the preferred embodiments and the accompanying drawings.
FIG. 1 is a schematic diagram of the flow of a preferred embodiment of the synthetic manufacturing method of a preferred Embodiment 1 of the present invention;
FIG. 2 is a schematic diagram of the flow of a preferred embodiment of the synthetic manufacturing method of a preferred Embodiment 2 of the present invention;
FIGS. 3A and 3B are the Electrostatic Potential Distribution (ESP) of each atom of the chemical structure corresponding to Embodiments 2-1 and 2-2, respectively;
FIG. 4 is a hydrogen Nuclear Magnetic Resonance (NMR) spectroscopy of Embodiment 2-1 of the present invention;
FIG. 5 is a coulombic efficiency and capacitance retention results of Embodiment 2-1 of the present invention and the Comparative Example applied to an anode-free lithium metal battery;
FIG. 6 is a cycle life performance of the Li||NMC lithium metal semi-battery applied to Embodiment 2-1 of the present invention; and
FIGS. 7A and 7B are scanning electron microscopes of the negative electrode surface of the Cu∥NMC battery corresponding to FIG. 5 after charging and discharging using Embodiment 2-1 of the present invention and the Comparative Example.

### DETAILED DESCRIPTION OF THE PREFERRED EMBODIMENTS

Reference will now be made in detail to the present preferred embodiments of the invention, examples of which are illustrated in the accompanying drawings. Wherever possible, the same reference numbers are used in the drawings and the description to refer to the same or like parts. It is not intended to limit the method by the exemplary embodiments described herein. In the following detailed description, for purposes of explanation, numerous specific details are set forth in order to attain a thorough understanding of the disclosed embodiments. It will be apparent, however, that one or more embodiments may be practiced without these specific details. As used in the description herein and throughout the claims that follow, the meaning of "a", "an", and "the" may include reference to the plural unless the context clearly dictates otherwise. Also, as used in the description herein and throughout the claims that follow, the terms "comprise or comprising", "include or including", "have or having", "contain or containing" and the like are to be understood to be open-ended, i.e., to mean including but not limited to.

The so-called dissolution or solvation of the present invention refers to a series of chemical reactions generated by the solvent in a chemical solution, which will affect the reaction rate, reaction equilibrium, solubility, stability, and even the reaction mechanism of the chemical reaction. Therefore, the present invention is an electrolyte solvent with adjustable properties for the overall reaction in the solution.

### <Electrolyte solvent with adjustable solubility properties

### Embodiment 1>

An electrolyte solvent with adjustable solvation properties of the present invention Embodiment 1 has a chemical structure formula shown in Formula (1) as follows: 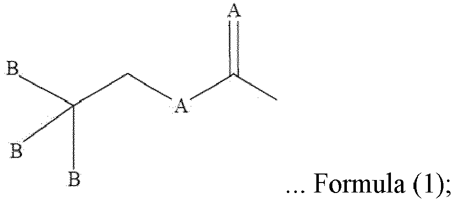 wherein A is oxygen (O), sulfur (S), selenium (Se), tellurium (Te) or combinations thereof; B comprises 1, 2 or 3 substituent(s) of halogen, nitrile (CN), hydrogen (H), nitro (NO₂), nitroso (NO), amine (NH₂) or combinations thereof.

The element not shown in Formula (1) is a carbon (C). The foregoing halogens include fluorine (F), chlorine (Cl), bromine (Br), iodine (I), or atom (At). The element not shown in Formula (1) is carbon (C), which is not specifically shown to be bonded to hydrogen (H), and which may be bonded to other elements or functional groups by carbon single bonds, carbon double bonds, or carbon triple bonds without affecting the properties of the present invention, without limitation herein.

### <Electrolyte solvent with adjustable solubility properties

### Embodiment 2>

An electrolyte solvent with adjustable solvation properties of the present invention Embodiment 2 has a chemical structure formula shown in Formula (2) as follows: 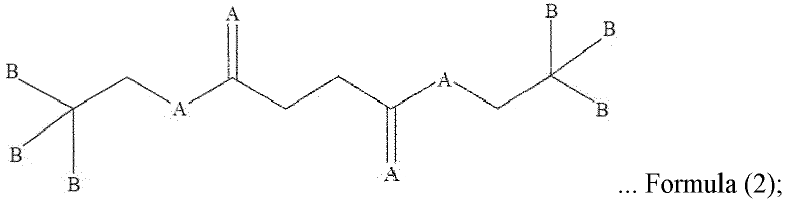 wherein A is oxygen (O), sulfur (S), selenium (Se), tellurium (Te), or combinations thereof; B comprises 1, 2 or 3 substituent(s) of halogen, nitrile (CN), hydrogen (H), nitro (NO₂), nitroso (NO), amine (NH₂), or combinations thereof.

The element not shown in Formula (2) is a carbon (C), which is not specifically shown to be bonded to hydrogen (H), and which may be bonded to other elements or functional groups by carbon single bonds, carbon double bonds, or carbon triple bonds without affecting the properties of the present invention, without limitation herein.

### <Electrolyte solvent with adjustable solubility properties

### Embodiment 3>

An electrolyte solvent with adjustable solvation properties of the present invention Embodiment 3 has a chemical structure formula shown in Formula (3) as follows: 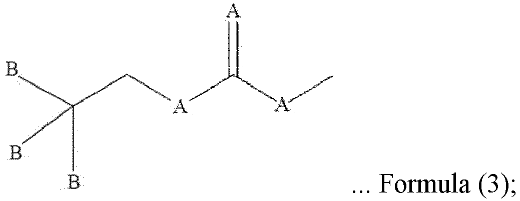 wherein A is oxygen (O), sulfur (S), selenium (Se), tellurium (Te), or combinations thereof; B comprises 1, 2 or 3 substituent(s) of halogen, nitrile (CN), hydrogen (H), nitro (NO₂), nitroso (NO), amine (NH₂), or combinations thereof.

The element not shown in Formula (3) is a carbon (C), which is not specifically shown to be bonded to hydrogen (H), and which may be bonded to other elements or functional groups by carbon single bonds, carbon double bonds, or carbon triple bonds without affecting the properties of the present invention, without limitation herein.

Referring to Table 1 below, it is illustrated that the electrolyte solvents with the aforementioned adjustable solvation properties are shown in Embodiment 2 and Embodiment 3, and the names of several preferred chemically structured embodiments. In the following embodiments, the element not shown is carbon (C), which is not specifically shown to be bonded to hydrogen (H), and which may be bonded to other elements or functional groups by carbon single bonds, carbon double bonds, or carbon triple bonds without affecting the properties of the present invention, without limitation herein.

**Table 1.**

| Embodiment | Chemical Formula | Name |
|---|---|---|
| Embodiment 2-1 | 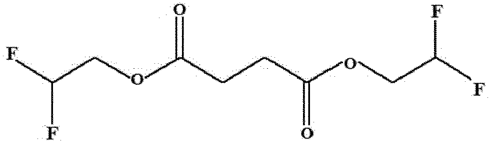 | Bis(2,2-difluoroethy 1) succinate (DFES) |
| Embodiment 2-2 | 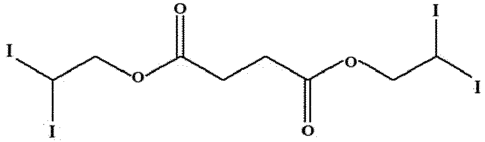 | Bis(2,2-diiodoethyl) succinate (DIES) |
| Embodiment 2-3 | 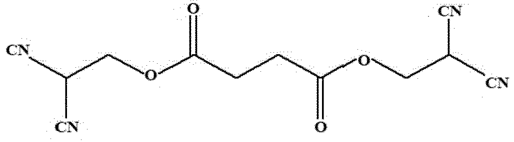 | Bis(2,2-dicyanoethyl ) succinate (DCES) |
| Embodiment 3-1 | 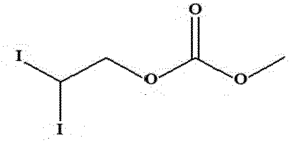 | 2,2-diiodoethyl methyl carbonate (DIEMC) |
| Embodiment 3-2 | 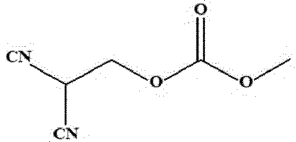 | 2,2-dicyanoethyl methyl carbonate (DCEMC) |

### <Production method of electrolyte solvent with adjustable solvation properties Embodiment 1>

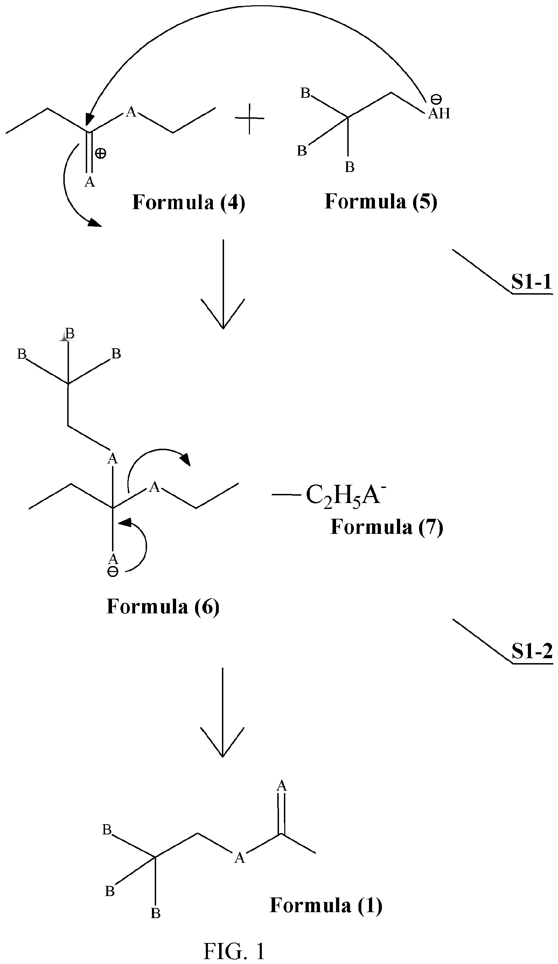

A preferred embodiment of the synthetic production method of the preferred Embodiment 1 of the present invention, the steps of which comprise:
Step S1-1), with reference to FIG. 1, reacting a chemical structure of Formula (4) with a chemical structure of Formula (5), wherein the negatively charged A⁻ in Formula (5) reacts with the positively charged double bond in Formula (4) to cause the positively charged double bond in Formula (4) to form a single bond, and causing the bonding of Formula (5) to Formula (4) to obtain the chemical structure as in a Formula (6); 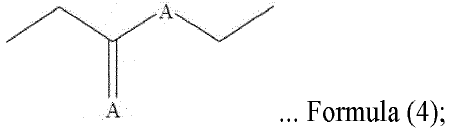 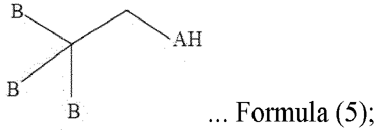 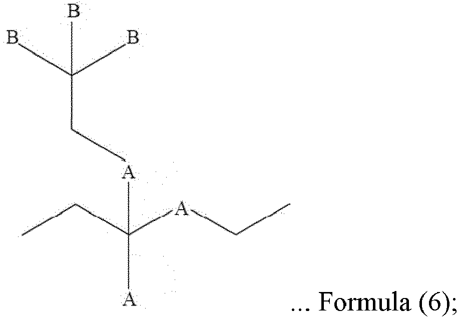
Step S1-2), the original Formula (5) and Formula (4) again form a double bond at the bonding point in Formula (6), and a chemical structure of Formula (7) is formed by removing an ethyl group and a functional group A⁻ to obtain the compound of the present invention as in the preferred Embodiment 1 of Formula (1).

C₂H₅A⁻...Formula (7). Wherein A is oxygen (O), sulfur (S), selenium (Se), tellurium (Te), or combinations thereof; B comprises 1, 2 or 3 substituent(s) of halogen, nitrile (CN), hydrogen (H), nitro (NO₂), nitroso (NO), amine (NH₂) or combinations thereof; and the element not shown at the single bond is carbon (C). The AH group in Formula (5) is a functional group formed by an element of the preceding code A with hydrogen (H).

### <Production method of electrolyte solvent with adjustable solvation properties Embodiment 2>

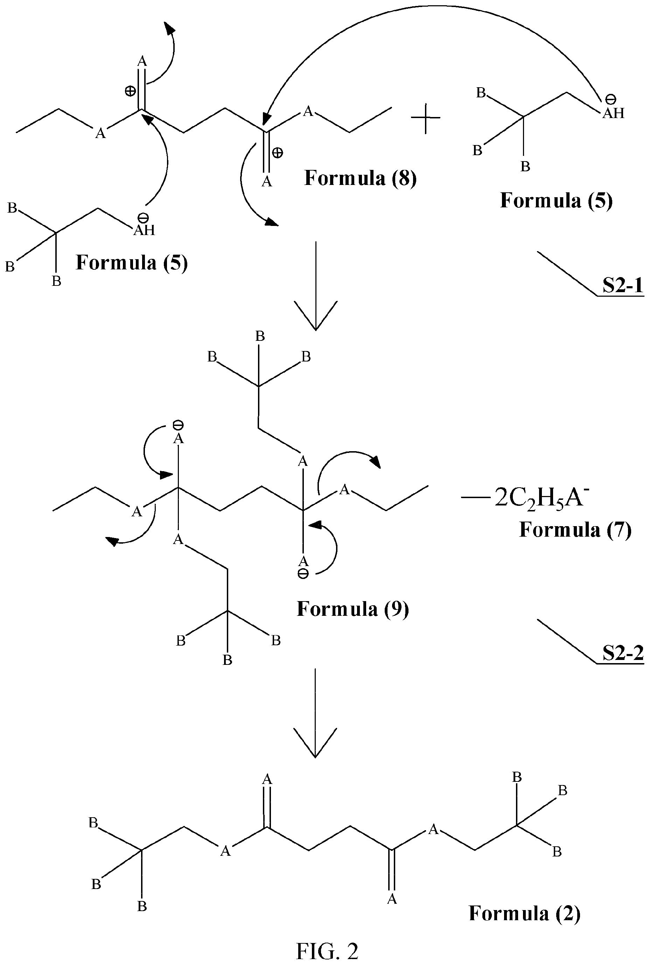

A preferred embodiment of the synthetic production method of the preferred Embodiment 2 of the present invention, the steps of which comprise:
Step S2-1), with reference to FIG. 2, reacting a chemical structure of Formula (8) with two chemical structures of Formula (5), wherein the two negatively charged A⁻ in Formulas (5) react with the two positively charged double bonds in Formula (8) to cause the two positively charged double bonds in Formula (8) to form a single bond, and causing the bonding of Formula (8) to Formulas (5) to obtain a chemical structure as in Formula (9); 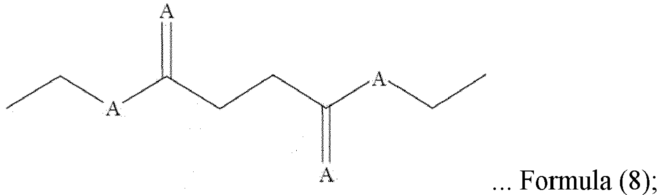 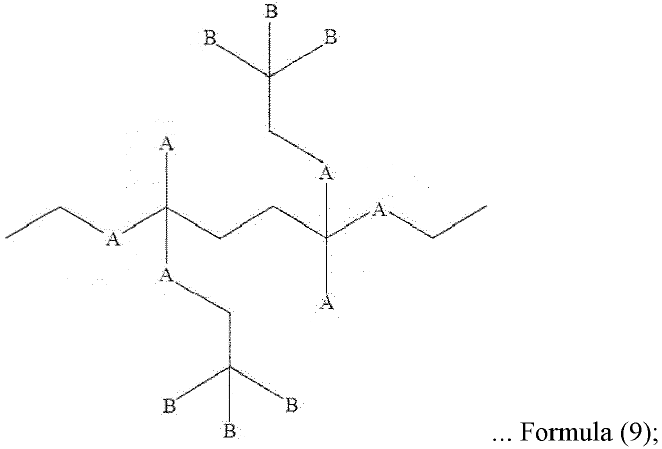
Step S2-2), the original Formula (8) and Formulas (5) again form a double bond at the bonding point in Formula (9), and the chemical structure of Formula (7) is formed by removing two ethyl groups and A⁻ to obtain the compound of the present invention as in the preferred Embodiment 2 of Formula (2).Similarly, in each chemical formula of this Embodiment, A is oxygen (O), sulfur (S), selenium (Se), tellurium (Te), or combinations thereof; B comprises 1, 2 or 3 substituent(s) of halogen, nitrile (CN), hydrogen (H), nitro (NO₂), nitroso (NO), amine (NH₂) or combinations thereof, and the element not shown at the single bond is carbon (C).

To practice the above mention preferred embodiment of production methods, the present invention could also obtain the embodiment by:
12 g DES, 10.05 g 2,2-difluoroethanol, 2.75 g triethylamine (TEA, Et3N) and 20 ml anhydrous acetone were added in to a 250 ml round bottom flask, and the solution was cooled to 0 °C by ice bath to stir for 10 min. The mixture was mixed with 10 ml anhydrous Dichloromethane (DCM) and was added dropwise into the flask. After completing the addition, the ice bath was removed to allow the suspension to warm up to room temperature. The reaction was stirred at room temperature for 48 h. After the completion of reaction, 5 ml deionized water was slowly added into the suspension to dissolve all solids. The DCM layer was separated and washed with brine, dried by anhydrous Magnesium sulfate (MgSO₄), and the solvents were removed under vacuum. A colorless liquid weighing approximately 19.62 g and yielding 89% was obtained by distilling the raw material three times at a temperature of about 80 °C. After the syntheses, the present invention were mixed with 10 wt% activated molecular sieves and stored in argon-filled glove box (Vigor, oxygen <0.5 ppm, water <0.1 ppm) at room temperature. The water contents of the present invention measured by Karl-Fisher titration were roughly 70 ppm.

### <Validation Test>

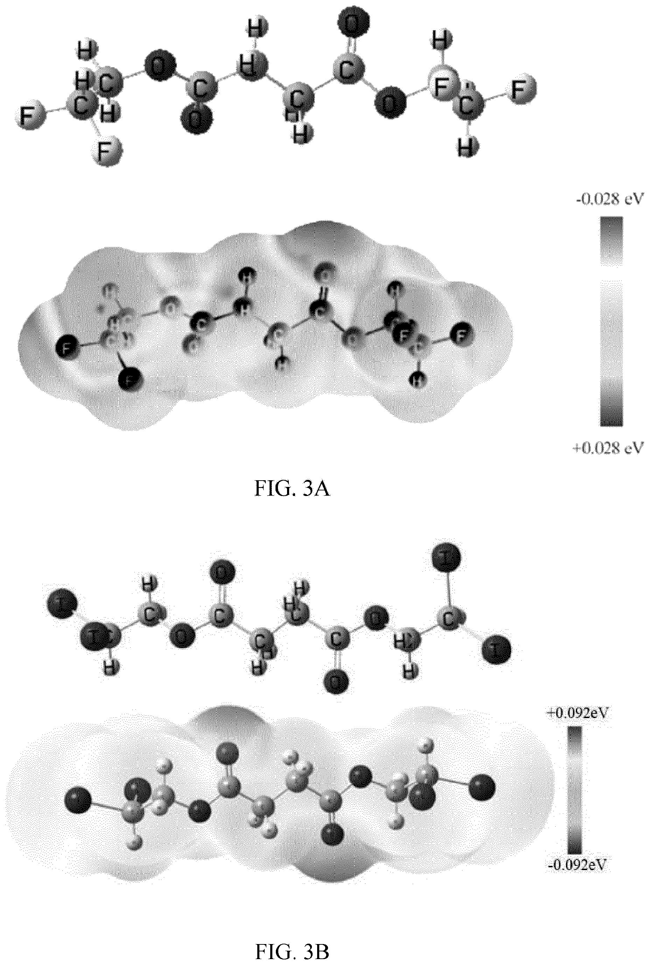

The electrolyte solvent with adjustable solvation properties provided by the present invention can be preferably used in electrochemical devices, such as the solvent for electrolytes in electrochemical batteries. Referring to FIGS. 3A and 3B are the Electrostatic Potential Distribution (ESP) diagrams for each atom of the chemical structure corresponding to Embodiments 2-1 and 2-2 in Table 1 above. The light and dark colored areas (red area in the colored diagram) in FIGS. 3A and 3B have negative potential positions, such as C=O and F, I, and the best positions are between C=O and F, I, which is the metal salt in the solvation and the electrolyte, such as the solvation coordination of lithium-ion (Li⁺) in the lithium salt, which can help the metal-ion to form a dense and conductive beneficial solid electrolyte interface (SEI) at the negative electrode and facilitate the metal deposition. The darkest area in FIG. 3 (blue area in the colored diagram) is a positive potential position, such as CH₃, which has a good interaction with the negative charge of the metal salts in the electrolyte, such as lithium salts, and helps stabilize the cathode electrode interface (CEI) at the positive electrode. Similarly, the cation (Li⁺) and the corresponding anion of other lithium salts can be stabilized in the positive and negative potentials of the solvent molecules of the present invention, respectively. Through the appropriate selection of A and B elements, the present invention can help to deposit metal on the negative electrode surface of the electrochemical device and form a beneficial solid electrolyte interface (SEI), and also interact well with the anions of the metal salt in the electrolyte to inhibit the formation of an undesirable cathode electrode interface (CEI) at the positive electrode.

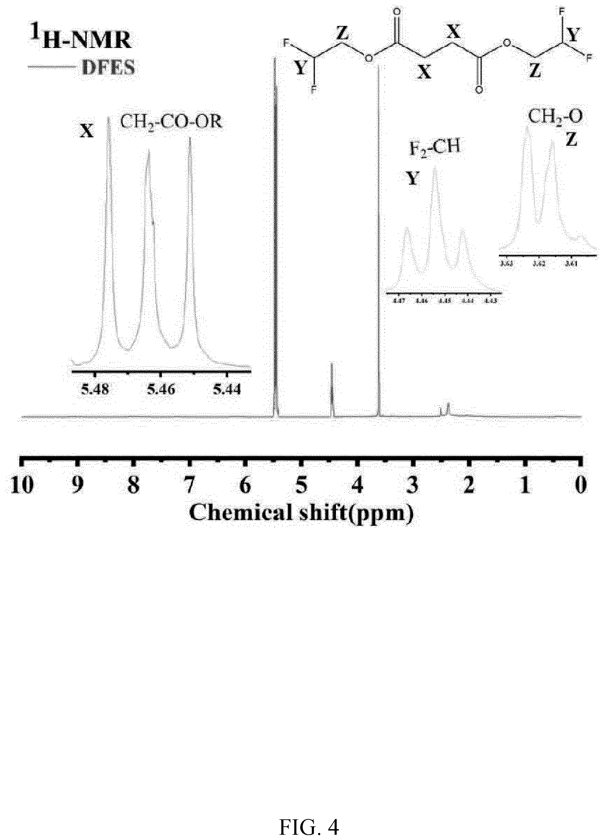

Referring to FIG. 4, which is the 1H-NMR spectrum of the present invention corresponding to Embodiment 2-1 (DFES) in the preceding Table 1, it can be seen from FIG. 4 that the chemical structures of the Embodiment of the present invention show corresponding chemical shift peaks for the structures marked X, Y, and Z. Therefore, it can be confirmed that the present invention is indeed successfully synthesized by the aforementioned synthetic preparation method and possesses the claimed chemical structures.

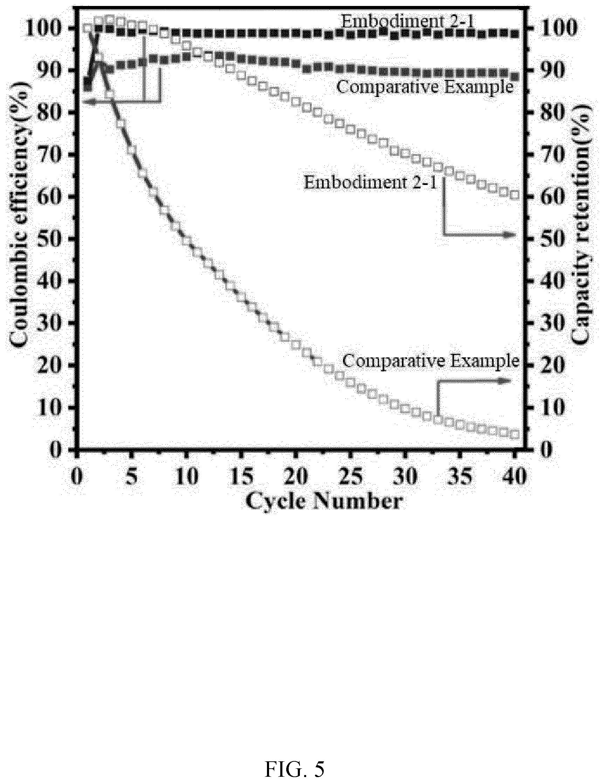

Referring to FIG. 5, the electrolyte solvent with adjustable solvation properties corresponding to Embodiment 2-1 in the preceding Table 1 and a solvent with ethylene carbonate (EC) and diethyl carbonate (DEC) as a Comparative Example were applied to the anode-free battery Cu∥NMC lithium metal battery, in which the lithium salt in the electrolyte was lithium bis(trifluoromethanesulphonyl)imide (LiTFSI), and tested with overcharge (4.5V) to obtain the results of coulombic efficiency and capacitance retention. As shown in FIG. 5, it can be seen that the average coulombic efficiency (ACE) of the Embodiment 2-1 still maintains nearly 100% after 40 cycles of charging and discharging, even under the more severe test conditions of overcharging, compared to the Comparative Example, which has dropped to less than 90%. The capacitance retention (CR) of the present invention Embodiment still remains significantly at least 55% after 40 cycles of charging and discharging, compared to the 0% capacity of the Comparative Example, indicating that the present invention is indeed able to maintain the electrical properties of the lithium metal battery.

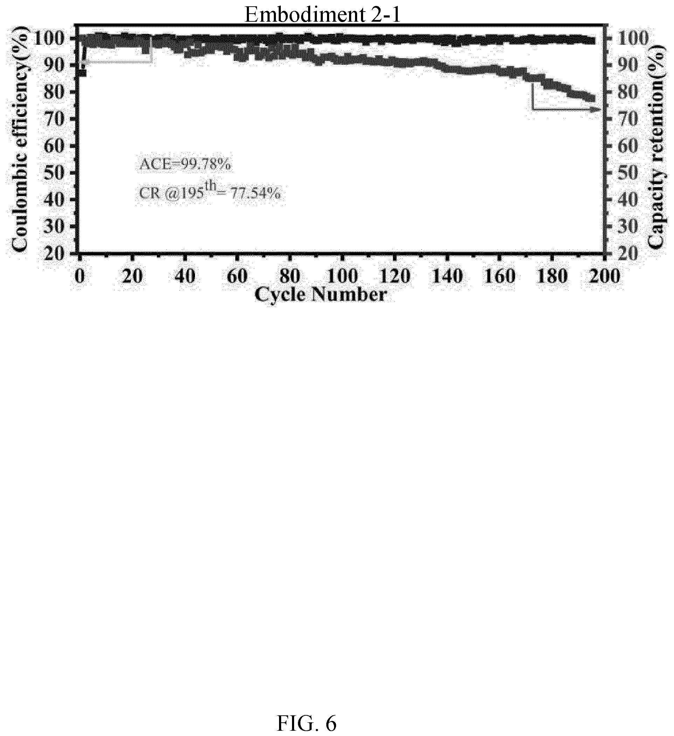

Referring to FIG. 6, the cycle life performance of Li∥NMC lithium metal semi-battery with the lithium salt of lithium bis(trifluoromethanesulphonyl)imide (LiTFSI) in the electrolyte has been applied to overcharge (4.5V) using the electrolyte solvent with adjustable solvation properties of Embodiment 2-1 of the present invention corresponding to the aforementioned Table 1. As can be seen from FIG. 5, under the severe conditions of 4.5V overcharge, Embodiment 2-1 still maintains 99.78% average coulombic efficiency (ACE) after 200 cycles and 77.54% capacitance retention (CR) at the 195^{th} cycle, indicating that the present invention can indeed extend or maintain the electrical properties of the battery.

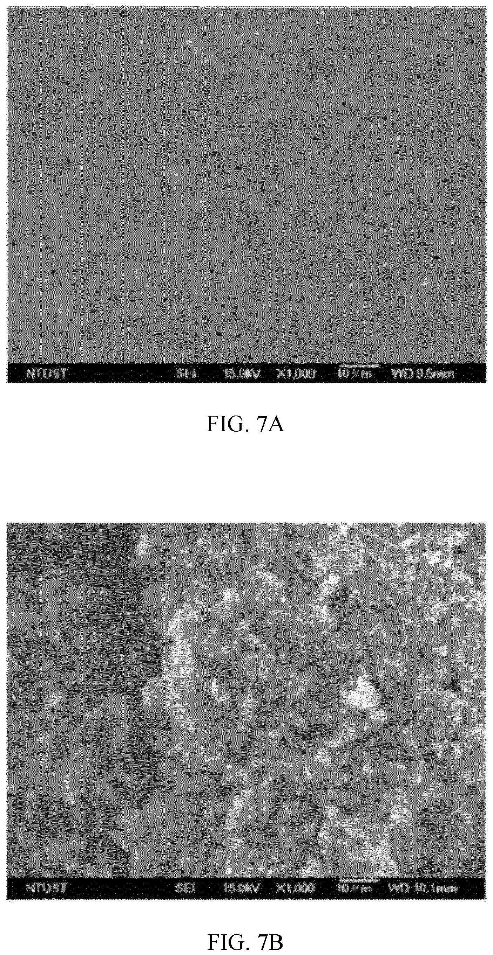

Referring to FIGS. 7A and 7B, which are scanning electron micrographs (SEM) of the negative electrode surface observed after charging and discharging using the electrolyte solvent with adjustable solvation properties of the present invention corresponding to Embodiment 2-1 in Table 1 above, and a solvent of ethylene carbonate (EC) and diethyl carbonate (DEC) as a Comparative Example, corresponding to the Cu∥NMC battery of FIG. 5 above. Wherein, FIG. 7A shows a flat and dense surface of the negative electrode of Embodiment 2-1, indicating the formation of a dense Li metal plating with solid electrolyte interface (SEI), which is beneficial to enhance the electrical properties and lifetime performance of the battery. On the contrary, the surface of the Comparative Example in FIG. 7B is rough and loose, indicating the formation of a large number of lithium dendrites or dead lithium, which cannot be used in the electrochemical reaction and will eventually reduce the electrical properties and lifetime performance of the battery.

### <Applications of electrolyte solvent with adjustable solvation properties>

The electrolyte solvent with adjustable solvation properties provided by the present invention can be preferably used in an electrochemical device, such as electrolyte solvent in electrochemical battery. The electrochemical battery that can be applied include a lithium battery, a sodium battery, a magnesium battery, a zinc battery, an aluminum battery, etc., and preferably include the aforementioned metal, metal-ion, and hybrid battery.

The above specification, examples, and data provide a complete description of the present disclosure and use of exemplary embodiments. Although various embodiments of the present disclosure have been described above with a certain degree of particularity, or with reference to one or more individual embodiments, those with ordinary skill in the art could make numerous alterations or modifications to the disclosed embodiments without departing from the spirit or scope of this disclosure.

## Claims

1. An electrolyte solvent with adjustable solvation properties, **characterized in that**: the electrolyte solvent comprises a chemical structure of Formula (1) as follows: 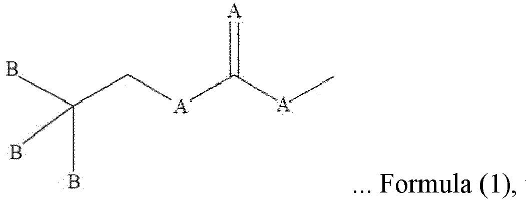 wherein each A is independently oxygen (O), sulfur (S), selenium (Se), or tellurium (Te); B comprises 1, 2 or 3 substituent(s) of halogen, nitrile (CN), nitro (NO₂), nitroso (NO), amine (NH₂), or combinations thereof.

2. An electrolyte solvent with adjustable solvation properties **characterized in that** the electrolyte solvent comprises a chemical structure of Formula (2) as follows: 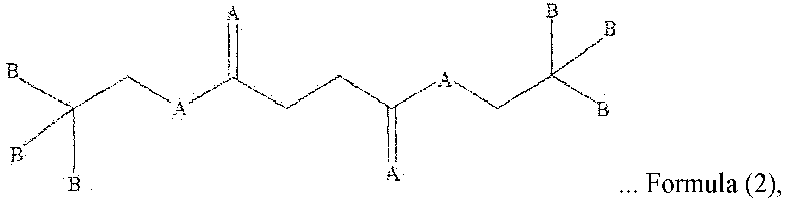 wherein each A is independently oxygen (O), sulfur (S), selenium (Se), or tellurium (Te); B comprises 1, 2 or 3 substituent(s) of halogen, nitrile (CN), nitro (NO₂), nitroso (NO), amine (NH₂), or combinations thereof.

3. A production method of the electrolyte solvent with adjustable solvation properties, **characterized in that**: the production method comprises the steps of:
the Formula (5) combines Formula (4) by a double bond and forms Formula (6) with removing an ethyl group and a functional group A⁻ to obtain the electrolyte solvent with adjustable solvation properties as described in Formula (1) of claim 1;
the original Formula (5) combines Formula (4) at the double bond and forms Formula (6).Formula (7) is produced by removing an ethyl group and A to obtain the electrolyte solvent with adjustable solvation properties as described in Formula (1) of claim 1; 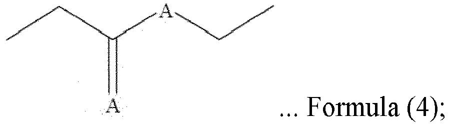 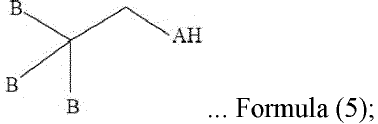 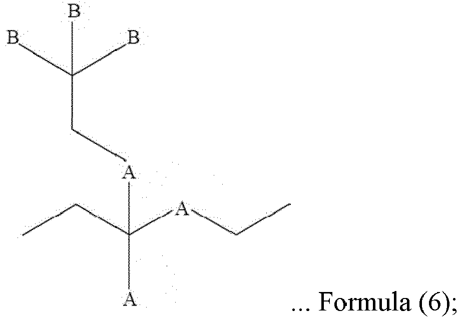
C₂H₅A⁻... Formula (7); wherein each A is independently oxygen (O), sulfur (S), selenium (Se), or tellurium (Te); B comprises 1, 2 or 3 substituent(s) of halogen, nitrile (CN), nitro (NO₂), nitroso (NO), amine (NH₂), or combinations thereof.

4. A production method of the electrolyte solvent with adjustable solvation properties according to claim 2, which comprises:
reacting a following chemical structure of Formula (8) with two chemical structures of Formula (5) as defined in claim 3, and bonding Formula (8) to Formulas (5) to obtain a chemical structure as in Formula (9);
the original Formula (8) and Formulas (5) again forming a double bond at the bonding point in Formula (9), and forming the chemical structure of Formula (7) as defined in claim 3 by removing two ethyl groups and A⁻ to obtain a chemical structure as described in Formula (2) of claim 2; 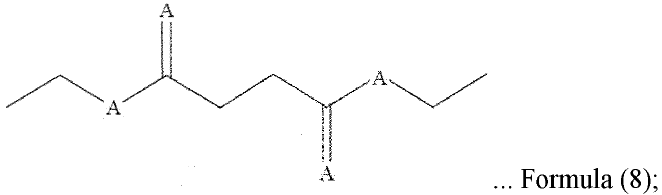 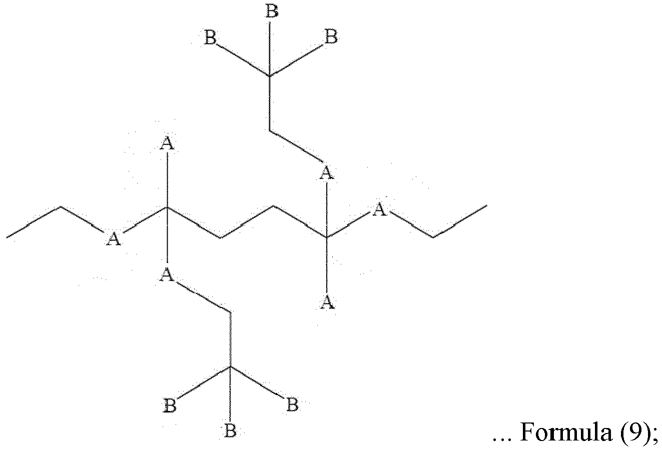 wherein each A is independently oxygen (O), sulfur (S), selenium (Se), or tellurium (Te); B comprises 1, 2 or 3 substituent(s) of halogen, nitrile (CN), nitro (NO₂), nitroso (NO), amine (NH₂), or combinations thereof.

5. An electrochemical device, **characterized in that**: it comprises the electrolyte solvent with adjustable solvation properties as described in claim 1 or 2.

6. The electrochemical device according to claim 5, wherein the electrochemical device includes a metal battery.

7. The electrochemical device according to claim 6, wherein the metal battery includes a lithium battery, a sodium battery, a magnesium battery, a zinc battery, or an aluminum battery.

8. The electrochemical device according to claim 6, wherein the metal battery includes a metal, a metal-ion, and a metal and metal-ion hybrid battery.

9. The electrochemical device according to claim 7, wherein the metal battery includes a metal, a metal-ion, and a metal and metal-ion hybrid battery.

10. The electrochemical device according to claim 5, wherein the electrochemical device comprises an anode-free battery.
